# EUROPEAN PATENT SPECIFICATION

(11) **EP 2 156 182 B1**
(45) Date of publication and mention of the grant of the patent: **06.10.2010**
(21) Application number: 08759013.9
(22) Date of filing: 04.06.2008
(51) Int. Cl.: G01N 33/50, G01N 33/538, B01D 15/34

(54) **DETECTION OF AN ANALYTE IN A SAMPLE OF HEMOLYZED WHOLE BLOOD**
DETEKTION EINES ANALYTEN IN EINER HÄMOLYSIERTEN VOLLBLUTPROBE
DETECTION D'UN ANALYTE DANS UN ECHANTILLON DE SANG TOTAL HEMOLYSE

(30) Priority: 06.06.2007 EP 07011169
(43) Date of publication of application: 24.02.2010
(73) Proprietor: Roche Diagnostics GmbH, 68305 Mannheim (DE); F. Hoffmann-La Roche AG, 4070 Basel (CH)
(72) Inventor: KOBOLD, Uwe, 82362 Weilheim (DE); DELMOTTE, Nathanael, CH-8004 Zurich (CH); HUBER, Christian, A- 5020 Salzburg (AT); VON DER ELTZ, Herbert, 82362 Weilheim (DE)
(86) International application number: PCT/EP2008/004457
(87) International publication number: WO 2008/148547

(56) References cited:
- EP-A- 0 185 870
- US-A- 4 056 468
- US-A- 5 292 663
- WAGNER K ET AL: "AN AUTOMATED ON-LINE MULTIDIMENSIONAL HPLC SYSTEM FOR PROTEIN AND PEPTIDE MAPPING WITH INTEGRATED SAMPLE PREPARATION" ANALYTICAL CHEMISTRY, AMERICAN CHEMICAL SOCIETY. COLUMBUS, US, vol. 74, no. 4, 15 February 2002 (2002-02-15), pages 809-820, XP001115830 ISSN: 0003-2700
- YANG ET AL: "Immunosuppressants: Pharmacokinetics, methods of monitoring and role of high performance liquid chromatography/mass spectrometry" CLINICAL AND APPLIED IMMUNOLOGY REVIEWS, ELSEVIER, AMSTERDAM, NL, vol. 5, no. 6, November 2005 (2005-11), pages 405-430, XP005289450 ISSN: 1529-1049
- BOOS K-S ET AL: "High-performance liquid chromatography integrated solid-phase extraction in bioanalysis using restricted access precolumn packings" TRAC, TRENDS IN ANALYTICAL CHEMISTRY, ELSEVIER, AMSTERDAM, NL, vol. 18, no. 3, March 1999 (1999-03), pages 175-180, XP004161231 ISSN: 0165-9936
- RIEUX ET AL: "Restricted-access material-based high-molecular-weight protein depletion coupled on-line with nano-liquid chromatography-mass spectrometry for proteomics applications" JOURNAL OF CHROMATOGRAPHY A, ELSEVIER, AMSTERDAM, NL, vol. 1149, no. 2, 26 April 2007 (2007-04-26), pages 169-177, XP022047973 ISSN: 0021-9673
- BOYER S H ET AL: "ENRICHMENT OF ERYTHROCYTES OF FETAL ORIGIN FROM ADULT-FETAL BLOOD MIXTURES VIA SELECTIVE HEMOLYSIS OF ADULT BLOOD CELLS: AN AID TO ANTENATAL DIAGNOSIS OF HEMOGLOBINOPATHIES" BLOOD, W.B. SAUNDERS, PHILADELPHIA, VA, US, vol. 47, no. 6, 1 June 1976 (1976-06-01), pages 883-897, XP000196837 ISSN: 0006-4971

## Description

The present invention relates to a method of detecting an analyte in a hemolyzed whole blood sample the method comprising the steps of applying a sample of hemolyzed whole blood known or suspected to contain an analyte of interest to a column comprising a restricted access chromatography material (RAM) thereby binding the analyte, eluting the analyte from the RAM and detecting the analyte, wherein at least in the first step a buffer with a pH above 8.0 is used. The novel method ensures that hemoglobin does not interfere with the detection of the analyte of interest. The method can be easily used in the online detection of many analytes, e.g. from a hemolyzed whole blood sample, like in the detection of an antibiotic, of folate or of immunosuppressive drugs, like tacrolimus or sirolimus.

### Background of the Invention

The more constituents are present in a sample the more difficult is the analysis of a target analyte comprised therein. Red blood cells contain a dramatic amount of proteins and small molecular weight constituents that potentially interfere with the detection of an analyte of interest from a biological fluid like whole blood. This is one of the major reasons why in clinical routine preferably blood plasma or simply referred to as plasma (i.e. an anticoagulated whole blood sample; deprived of cells and erythrocytes) or blood serum or referred to simply as serum (i.e. coagulated whole blood; deprived of cells, erythrocytes and most proteins of the coagulation system, especially fibrin/fibrinogen), respectively, are used. Whole blood samples also tend to be more difficult to handle, e.g., as compared to serum or plasma. Whole blood tends to be less stable and slow rupture of erythrocytes impairs a reliable measurement of quite a few analytes of interest.

As indicated above, serum or plasma may be obtained from whole blood and used in the detection of an analyte. Cells and erythrocytes in theory may also be removed by filtration or centrifugation from whole blood. However, these methods are neither appropriate for use in a routine diagnostic setting, nor would they allow for a correct measurement of those analytes that are at least partially present inside red blood cells.

The vast majority of procedures known in the art for the detection of an analyte from a whole blood sample require a further processing of the sample before the analyte can be quantified. In many procedures the analyte of interest is first separated from the majority of potentially interfering substances by selective precipitation or extraction methods. Extraction can be performed in liquid phase or on a solid phase. This shall be exemplified by illustrating some of the procedures used in the detection of some immunosuppressive drugs or folate, respectively.

Well-known immunosuppressive drugs are e.g. mycophenolate mofetil (MMF), rapamycin (RAPA also known as sirolimus) and tacrolimus (FK-506). Therapeutic drug monitoring for immunosuppressive drugs is especially important for transplant patients as well as for patients suffering from AIDS (cf. e.g.: Drug. Ther. Perspect. 17(22) (2001) 8-12). Most patients who undergo solid organ transplantation require lifelong immunosuppressive therapy to prevent allograft rejection. But, because many immunosuppressive agents have narrow therapeutic ranges, and are associated with various toxicities and the potential for drug interactions, the use of therapeutic drug monitoring (TDM) in conjunction with clinical assessment of patients may be particularly important.

Tacrolimus is a macrolide antibiotic that was first approved by the US Food and Drug Administration (FDA) in 1994 for the prevention of liver allograft rejection. It is up to 100 times more potent than cyclosporin *in vitro,* and clinically, it is associated with a greater reduction in the incidence of tissue rejection. Tacrolimus has demonstrated efficacy both as primary immunosuppressive therapy in patients undergoing various transplantation procedures, and as rescue therapy for patients with refractory acute allograft rejection after liver or kidney transplantation. The therapeutic trough concentration is in the range of 5-20 µg/L.

Since at least part of the tacrolimus present in the circulation is compartmented within erythrocytes, a whole blood sample is used in the clinical routine measurement of this drug. Tacrolimus can e.g. be detected by high performance liquid chromatography (HPLC), HPLC interfaced to mass spectrometry (MS), radio receptor assay (RRA), or by an immunoassay (IA). The latter two methodologies do not detect tacrolimus and certain of its various metabolites with the same sensitivity. This may lead to an interference in the procedure used (Murthy, J. N., et al., Clin. Biochem. 31 (1998) 613-617). At least in the detection of the various tacrolimus metabolites the HPLC-MS-procedure may be considered the gold standard. All the procedures mentioned above, however, require the extraction of tacrolimus from whole blood. Usually acetonitrile is used in clinical routine for the extraction of tacrolimus from whole blood and no method appears to exist that would allow for an online measurement of tacrolimus from a whole blood sample. Sirolimus is, like tacrolimus, a macrolide antibiotic. It was first approved in 1999 by the US FDA for the prevention of allograft rejection after kidney transplantation, and indeed has shown promising results in this respect when used acutely in combination with cyclosporin and corticosteroids. *In vitro,* sirolimus is up to 100 times more potent than cyclosporin, and clinically, it may exhibit synergism with cyclosporin, perhaps permitting a reduction in cyclosporin dosage. The therapeutic trough concentration is in the range of 5-15 µg/L.

As for tacrolimus, a significant amount of sirolimus is present within erythrocytes. Therefore extraction of a whole blood sample is required no matter which detection method is used. In clinical routine a sample suspected to comprise sirolimus is subjected to HPLC and sirolimus is detected by ultraviolet light (UV) or by tandem mass spectrometry (MS/MS). Recently also a microparticle enzyme immunoassay has been described (Jones, K., et al., Clinical Therapeutics 22, Suppl. B (2000) B49-B61).

Folate is the collective name of a group of related molecules differing in oxidation state. Folates are part of the water-soluble vitamin B group and are important as coenzymes for homocysteine metabolism and in the transfer of one-carbon groups required for DNA replication. Inadequate folate status is related to increased risk of neural tube defects, is associated with cardiovascular disease, anemia, with certain cancers and with Alzheimer's disease. Serum or plasma folate concentrations reflect recent dietary intake, whereas erythrocyte folate concentrations are more indicative of body stores (Gunter, E.W., et al., Clin. Chem. 42 (1996) 1689-1694; Fazili, Z., et al., Clin. Chem. 51 (2005) 2318-2325; Pfeiffer, C.M., et al., Clin. Chem. 50 (2004) 423-432). Erythrocyte total folate (red blood cell folate = RBC-folate) is the best measure of whole body folate status. Recent studies have shown that 5-methyl tetrahydrofolate is the dominant folate vitamer in circulating erythrocytes. For the diagnosis of folate deficiency it is recommended that determinations are performed not only from serum or from plasma but also from erythrocytes, since folate is localized to more than 95% in the latter. The concentration in the erythrocytes more truly reflects the actual folate status.

A number of methods are available to measure folate in different matrices. The major analytical methods are microbiological assay, radio immuno assay, chemiluminescence, chromatographic methods and mass spectrometric methods. Most methods are based on competitive binding of folate to folate binding protein. For the measurement of RBC-folate the use of a hemolyzing reagent is obviously mandatory. For example the Elecsys™ assay (Elecsys is a trademark of a member of the Roche Group) for determination of RBC folate uses ascorbic acid as lysis reagent. Elecsys RBC-folate hemolyzing reagent is used together with the Elecsys folate assay for the quantitative determination of folate in erythrocytes (RBC-folate). Whole blood treated with anticoagulants (heparin or EDTA) is diluted with ascorbic acid solution (0.2%) and incubated for approximately 90 minutes at 20-25°C. Lysis of the erythrocytes takes place, with liberation of the intracellular folate. The hemolysate is then used as a "prediluted" sample (in analogy to serum) for subsequent measurement in the Elecsys folate assay. The hematocrit value determined in whole blood and the dilution effect brought about by pretreatment of the sample is compensated for in the calculation of the erythrocyte folate concentration (Greiling, H., Gressner, A.M., Lehrbuch der Klinischen Chemie und Pathobiochemie, 3rd ed., Stuttgart-New York, Schattauer (1995), pp. 460-462; Gunter, E.W., et al., Clin. Chem. 42(1996) 1689-1694).

The hemolysate generated by treatment with ascorbic acid can not be used for routine chromatographic procedures, because of interfering substances contained therein. For use of such hemolysate in a chromatographic procedure or mass spectrometric determination it would necessary to remove cell debris and precipitated protein prior to analysis.

Debris and precipitated proteins usually are removed from a sample by centrifugation, offline filtration or solid phase extraction.

Solid phase extraction (SPE) is a chromatographic technique which is widely used, e.g., for preconcentration and cleanup of analytical samples, for purification of various chemicals, and for removal of toxic or valuable substances from aqueous solutions. SPE is usually performed using a column or cartridge containing an appropriate resin. SPE procedures have been developed using sorbents which can interact with analytes by hydrophobic, ion exchange, chelation, sorption, and other mechanisms, to bind and remove the analytes from fluids. Since different SPE applications for different classes of analytes can require different sorbents, there is a concomitant need for sorbents with specific properties which have unique selectivities for the analyte or class of analytes of interest. Representative examples of SPE materials and SPE columns, respectively, can be found in US 6,322,695 and US 6,723,236.

As obvious from the above discussion of the state-of-the-art procedures, the direct, especially the online measurement of these analytes from whole blood is not possible at all or at least suffers from complicated and/or time-consuming handling steps. Without wanting to be bound to the following theory, it may well be that the lack of appropriate procedures in the art is due to at least two reasons: a) a sample of hemolyzed whole blood often contains aggregates or precipitates and b) the extremely high concentration of hemoglobin interferes with the detection method.

The inventors of the present invention have addressed and solved both problems. First a method has been developed called differential hemolysis that allows e.g. for the complete lysis of the erythrocytes as contained in a sample of whole blood without formation of interfering aggregates or precipitates. The procedure for differential hemolysis is described in some detail below.

The inventors of the present invention, however, also discovered that the high concentration of hemoglobin as obviously present in a hemolyzed blood sample tends to interfere with the sensitive detection of an analyte of interest. This has even been observed when using the most advanced chromatography materials, the so-called restricted access chromatography materials (RAMs). If a sample of hemolyzed blood is applied to a column comprising a RAM according to standard procedures hemoglobin is not completely removed, rather hemoglobin tends to bind to the RAM column and thus tends to interfere in the analyte detection.

The inventors of the present invention thus were facing the problem that hemoglobin as e.g. present in a hemolyzed blood sample - even after differential hemolysis - may interfere with the detection of an analyte of interest. As obvious to the skilled artisan this problem becomes the more pronounced the more sensitive and the more precise the measurement has to be.

It would, however, be highly desirable if whole blood could be used directly as a sample, especially in the detection of low molecular weight analytes. This would in addition be especially advantageous in an online detection procedure making use of a liquid chromatography (LC) separation step. It is also obvious that e.g. the direct online detection of an immunosuppressive drug from whole blood would be an important progress for a clinical routine laboratory.

It has now surprisingly been found and could be established that it is possible to efficiently avoid interference by hemoglobin, e.g. by hemoglobin as comprised in a sample of hemolyzed whole blood. It has been found and is described in detail below that under special buffer conditions hemoglobin does not bind to RAMs, or at least does not interfere with an online detection of the analyte of interest. Under these conditions RAMs can be used in an elegant way in the detection of an analyte of interest from a hemolyzed whole blood sample.

### Summary of the Invention

In a first embodiment the present invention relates to a method of detecting an analyte in a hemolyzed whole blood sample the method comprising the steps of applying the sample of hemolyzed whole blood known or suspected to contain the analyte of interest to a column comprising a restricted access chromatography material (RAM) thereby binding the analyte, eluting the analyte from the RAM and, detecting the analyte, wherein at least in the step of applying the sample to the RAM a buffer with a pH above 8.0 is used.

In preferred embodiments it is e.g. described that the RAM is selected from porous silica or porous polymer-based particles and may have certain advantageous modifications of the inner and/or outer surface. The method described herein can be used in the detection of certain clinically important analytes.

### Detailed Description of the Invention

The method according to the present invention is performed in vitro, i.e. not on the human or animal body.

In a preferred embodiment the present invention relates to a method of detecting an analyte in a hemolyzed whole blood sample the method comprising the steps of a) applying the sample of hemolyzed whole blood known or suspected to contain the analyte of interest to a column comprising a restricted access chromatography material (RAM) thereby binding the analyte, b) eluting the analyte from the RAM and, c) detecting the analyte, wherein at least in step (a) a buffer with a pH above 8.0 is used.

Unless the contest dictates otherwise, the articles "a" and "an" are used herein to refer to one or to more than one (i.e. to at least one) of the grammatical object of the article. By way of example, "an analyte of interest" means one analyte of interest or more than one analyte of interest.

A "restricted access material" or restricted access chromatography material (RAM) according to the present invention is a chromatography material having pores with an inner surface capable of binding the analyte of interest and a pore size appropriate to prevent the protein fraction of the hemolyzed blood from entering into said pores.

The restricted access materials as originally described (Boos, K.S. and Rudolphi, A., LC-GC 15 (1997) 602-611, and Rudolphi, A. and Boos, K.S., LC-GC 15 (1997) 814-823) have a hydrophobic inner surface that works alike a reversed phase chromatographic material. The chromatography based on such RAM can be considered to be a combination of a reversed-phase chromatography and size exclusion chromatography.

The various types of RAM available are known to the skilled artisan.

Preferably the RAM used in a method according to the present invention is selected from porous silica or polymer-based particles. The RAM particles used in a method according to the present invention are in addition characterized by a hydrophilic coating.

The hydrophilic coating of the RAM, i.e. of the RAM outside of the pores is preferably based on a coating material providing hydroxyl groups like alkyldiol, carboxylic groups like carboxymethyl, amino groups like aminopropyl or bis(hydroxyethyl)aminoethyl, respectively. Also preferred is the use of hydrophilic polymers like polyethyleneglycol, polyvinylalcohol, oligo- and polysaccharides, dextran, peptides or proteins, respectively, to render the RAM outside the pores hydrophilic. Commercially available and preferred examples of RAM materials that can be used in a method as disclosed herein are Biotrap 500 MS^{®} (Chromtech, Cogleton, United Kingdom) or LiChrosphere^{®} RP-18 ADS (Merck, Darmstadt, Germany).

The inner surface of the RAM is capable of binding the analyte of interest. The inner surface of the RAM used in a method according the present invention will be chosen by the skilled artisan to match the chemical properties of the analyte of interest. The binding can be achieved by any appropriate interaction, like molecular imprinting, hydrophobic interaction, ionic interaction, or polar interaction. The skilled artisan is fully familiar with these types of interaction.

In a preferred embodiment the RAM used in a method according to the present invention is binding the analyte of interest by hydrophobic interaction or by polar interaction.

Preferably the inner pores of silica or polymer-based particles used in a method according to the present invention are coated with groups selected from anion or cation exchange materials or from hydrophobic groups, respectively.

Preferably the inner pores of silica or polymer-based particles used in a method as disclosed herein have a hydrophobically coated inner surface of the pores wherein the binding material is selected from the group consisting of aliphatic groups, phenyl groups, hydrophobic peptides or other reversed phase materials. Preferably the aliphatic group has between 4 and 20 C-atoms. Also preferred, the inner surface is a C4-, C8- C12-, C16- or C18-matrix. Preferred materials have a C4, C8 or a C18 coating of the inner surface.

The pore size is selected in such a way that the analyte(s) can enter the pores and therefore are bound to the inner surface of the pores, e.g. to the reversed phase. Ideally, polymeric matrix constituents, typically blood proteins, especially hemoglobin, are a) excluded from the pores by appropriate selection of the pore size and b) do not stick to the hydrophilic outer surface. If these requirements are met the polymers are not retained by the RAM and pass through the column in the void volume. In addition, since these polymers cannot enter the pores, almost no surface fouling which could alter the retention characteristics of the material, occurs. Generally, the analyte separation is performed on a second column, which is filled with a standard RP material. In a preferred embodiment the pore size of the RAM used in a method according to the present invention is between 60 and 120 A, also preferred between 60 and 100 Å.

RAMs are supposed for use in on-line extraction procedures wherein the analyte of interest is present in a very difficult matrix. However, it is desired and has proven necessary to remove as many proteins from a hemolyzed blood sample as possible in order to minimize any interference of such molecules with the measurement of an analyte. Dependent on the pore size selected it is possible to exclude proteins above a certain molecular weight. Preferably the protein fraction excluded comprises the polypeptides of 20 kDa and larger. Also preferred the protein fraction excluded comprises polypeptides of 19 kDa and larger, 18kDa and larger, 17kD and larger, 16kD and larger, and 15 kDa and larger.

The lower limit for size exclusion of commercially available RAM materials is given by the respective manufacturers as being about 15 to 20 kDa. Whereas RAMs work quite well with polypeptides having an apparent molecular weight of 20 kDa or more, hemoglobin - which has a molecular weight of only 16 kDa - represents a significant problem with the commercially available RAMs investigated. In fact this problem may be one of the reasons, or even the most important reason, why the online chromatographic detection of an analyte from a sample of hemolyzed blood still is not established in clinical diagnostic routine.

As can be seen from the experiments presented further below, hemoglobin unspecifically binds for example to a commercially available RAM ADS^{®} column, if this column is used according to a standard protocol. Upon elution of the bound materials, including the analyte of interest, hemoglobin is also set free from the RAM and interferes with the detection of the analyte. Surprisingly it has been found that the non-specific binding of hemoglobin, e.g. as contained in a sample of hemolyzed whole blood can be drastically reduced if the sample comprising hemoglobin is applied in a buffer having a pH of above 8.0.

"Applying" the sample at a certain pH to a RAM does not necessarily mean that the sample has this pH or has been adjusted to this pH, but means that the sample application buffer into which the sample is injected has this pH. The skilled artisan often refers to such buffer also as the first eluent or as buffer (A). In a routine and preferred mode of liquid chromatography the RAM column usually is equilibrated with the application buffer and the system is running, i.e. the application buffer is flowing through the chromatography setup and the sample is injected into the buffer stream. This is why the application buffer can also be referred to as an eluent, e.g. as eluent (A). As the skilled artisan will appreciate the buffering capacity of the application buffer will be chosen to match the buffer strength of the sample. In case of a hemolysate the molarity of the buffer can be as low as 5mM. Preferably the buffer will be used at a concentration of about 10 or of about 20mM. As indicated, the buffer strength can be easily selected by the skilled artisan to match the requirements.

At a pH above 8.0 the non-specific binding of hemoglobin to a RAM is abolished or reduced to a level that does not interfere with the detection of an analyte of interest, respectively. Preferably the pH of the buffer used to apply the sample to the RAM is at least pH 8.5 or higher or also preferred at least pH 9.0 or higher, or pH 9.5 or higher. The high end of the pH-range can be selected as appropriate. The skilled artisan will choose a pH that does neither destroy the analyte of interest nor the RAM used. Preferably the pH at the high end will be pH 12.5 or less. Also preferred it will be pH12.0 or less, or pH 11.5 or less, or pH 11.0 or less, respectively.

In an elegant and preferred online setup, the sample is applied to a RAM under appropriate buffer conditions and the RAM is washed with an appropriate washing buffer. By adjusting the valves and flow direction of the system, the analyte - if present - is eluted, passed over an analytical column and detected. In a further preferred embodiment the method of the present invention is practiced by using the same pH for application and elution buffer. As the skilled artisan knows the elution of an analyte of interest can be adjusted to meet the conditions most appropriate for the analyte under investigation. In many cases a gradient elution will serve the needs.

An analyte according to the present invention may be any inorganic or organic molecule, including a biomolecule, excluding nucleic acids. The analyte will not be a nucleic acid, especially it will not be a DNA. Preferably the analyte is selected from the group consisting of a polypeptide, a carbohydrate, and an inorganic or organic drug molecule. Preferably the analyte of interest has a molecular weight of 10 kDa or below, also preferred of 9 kDa or below, of 8 or below, of 7 kDa or below, of 6 kDa or below, or of 5 kDa or below, respectively.

A polypeptide or protein is a molecule that is essentially composed of amino acids and that has at least two amino acids linked by peptidic linkage. In case the analyte of interest to be investigated in a method disclosed here, the polypeptide preferably will consist of at least 3, 4, 5, 6, 7, 8, 9, 10, 12, 15, 20, 25, and 30 to up to about 100 amino acids. Preferably the polypeptide will contain from 5 to 100, also preferred from 10 to 40 amino acids. Suitable peptidic analytes of interest are e.g. peptide hormones, and other polypeptides present in the circulation and especially polypeptides released from red blood cells due to the treatment with a membrane solubilizing agent according to the present invention.

Preferably the method according to the present invention is used in the online detection of an analyte from a whole blood sample wherein said analyte is at least partially located inside a red blood cell, like sirolimus, tacrolimus or folate.

A preferred target analyte according to the present invention is selected from the group consisting of the drugs of abuse and the immunosuppressive drugs. Preferred target analytes are the drugs of abuse. The drug of abuse is preferably selected from the group consisting of amphetamine, cocaine and cocaine metabolites like benzoylecgnonine, methamphetamine, opiate and opiate derivatives, cannabinoids like tetrahydrocannabinol, and phencyclidine.

Another preferred target analyte is an immunosuppressive drug. The immunosuppressive drug is preferably selected from the group consisting of cyclosporine (CsA), mycophenolate mofetil (MMF), rapamycin (RAPA also known as sirolimus), tacrolimus (FK-506) azathioprine (AZA), and methylprednisolone (MP).

A further preferred target analyte is folic acid or a folic acid vitamer, respectively. One preferred analyte is the total folate as comprised in both the blood plasma and in the red blood cells.

In the method according to the present invention the analyte of interest is first bound to a RAM under appropriate conditions. For detection and/or quantitation the analyte is eluted from the RAM and thereafter detected and or quantified by an appropriate method. Preferably the analyte of interest is eluted from the RAM and thereafter separated by aid of liquid chromatography. Preferably the material eluted from the RAM is further separated by reversed phase HPLC

Liquid chromatography (LC) is an extremely important analytical technique which is used for the separation, identification and quantitation of an analyte of interest even if present in a complex mixture of different sample constituents. During LC the chemical components in a mixture are carried through a column bed packed with a suitable stationary phase by the flow of a liquid mobile phase. The stationary phase, usually irregularly or spherical particles, have a surface that is suitable for reversible interactions with the analytes. The particles may be porous in order to increase the surface area available for interactions. Separation in liquid chromatography is achieved by means of differences in the interactions of the analytes with both the mobile and stationary phases. As the skilled artisan appreciates both a stationary phase and a mobile phase appropriate to the analytes under investigation have to be chosen. In addition, the user will identify chromatographic conditions appropriate to maintain the sharpness of analyte bands as a sample moves through the stationary phase column to the detector.

High Performance Liquid Chromatography, also known as High Pressure Liquid Chromatography, abbreviated as HPLC, is a special form of liquid chromatography and nowadays used frequently in biochemistry and analytical chemistry. Compared to LC, the particle size of the stationary phase is smaller, which, according to well known theory, results in less band dispersion and narrower peaks in the resulting chromatogram and thence to better resolution and sensitivity. Because of the smaller particle diameter, the mobile phase in an HPLC application has to be forced through a column at high pressure.

The mobile phase is chosen to ensure solubility of the sample solutes. For the stationary phase, preferably microparticulate silica (bare or chemically modified) is used, because its high surface area accentuates the differences in solute-stationary phase interactions. The use of a stationary phase that interacts strongly with solutes relative to solute mobile-phase interactions will result in very long retention times, a situation which is not analytically useful. Hence the stationary phase must be selected so as to provide weak to moderate solute interactions relative to those in the mobile phase. As a consequence, the nature of the solute governs the type of LC selected. The stronger interactions should occur in the mobile phase to ensure sample solubility and ready elution, while the stationary phase should be responsive to more subtle differences among the solutes. For example, polar neutral compounds are usually better analyzed using a polar mobile phase together with a nonpolar stationary phase that distinguishes subtle differences in the dispersive character of the solutes. One of the powerful aspects of HPLC is that the mobile phase can be varied to alter the retention mechanism. Modifiers can be added to the mobile phase to control retention. For example, pH is an important variable in aqueous mobile phases.

Five general classes of LC can be distinguished:
1. Normal-phase chromatography calls for the use of a polar stationary phase in conjunction with a non-polar (dispersive) mobile phase.
2. Reversed-phase chromatography, the opposite possibility, calls for the use of a non-polar stationary phase and a polar mobile phase (composed of one or more of the polar solvents, e.g. water, methanol, acetonitrile, and tetrahydrofuran).
3. Ion-exchange chromatography involves ionic interactions. In this case the mobile phase must support ionization to ensure solubility of ionic solutes. The stationary phase must also be partially ionic to promote some retention.
4. Size-Exclusion chromatography involves separations based on molecular size alone and ideally requires that there be no adsorptive interaction of the solutes with the stationary phase.
5. Affinity chromatography is based on a specific interaction, e.g. between the members of a specific binding pair, like antigen and corresponding antibody or receptor and corresponding ligand. For example a first partner of a binding pair is bound to an appropriate stationary phase and used to capture the second partner of the binding pair. The second partner can be released and isolated by appropriate means.

The general classification of separation principles given above must not be exhaustive and therefore is non-limiting, there are other separation principles which can be used for the separation of liquid samples, e.g. hydrophobic interaction chromatography, hydrophilic interaction chromatography, ion-pair reversed-phase chromatography, molecular imprinted materials based separation.

The characterization and or quantification of an analyte of interest can be performed by any appropriate method. Appropriate and preferred detectors sense the presence of a compound passing through, and provide an electronic signal to a recorder or computer data station. The output is usually in the form of a chromatogram and a substance of interest is usually found in a certain peak. The peak area or peak height can be used to quantify the amount of analyte present in the sample investigated.

The detector for an HPLC system is the component that emits a response due to the eluting sample compound and subsequently signals a peak on the chromatogram. It is positioned immediately posterior to the stationary phase in order to detect the compounds as they elute from the column. The bandwidth and height of the peaks may usually be adjusted using the coarse and fine tuning controls, and the detection and sensitivity parameters may also be controlled by the skilled artisan. There are many types of detectors that can be used with HPLC. Some of the more common detectors include: Refractive Index (RI), Ultra-Violet (UV), Fluorescence, Radiochemical, Electrochemical, Near-Infra Red (Near-IR), Mass Spectrometry (MS), Nuclear Magnetic Resonance (NMR), and Light Scattering (LS).

Refractive Index (RI) detectors measure the ability of sample molecules to bend or refract light. This property for each molecule or compound is called its refractive index. For most RI detectors, light proceeds through a bi-modular flow-cell to a photodetector. One channel of the flow-cell directs the mobile phase passing through the column while the other directs only the mobile phase. Detection occurs when the light is bent due to samples eluting from the column, and this is read as a disparity between the two channels.

Fluorescence detectors measure the ability of a compound to absorb then re-emit light at different wavelengths. Each compound has a characteristic fluorescence. The excitation light passes through the flow-cell while a monochromator and photodetector measure the emitted light intensity.

Radiochemical detection involves the use of radiolabeled material, usually tritium (³H) or carbon-14 (¹⁴C). It operates by detection of fluorescence associated with beta-particle ionization, and it is most popular in metabolite research.

Electrochemical detectors measure compounds that undergo oxidation or reduction reactions. This is usually accomplished by measuring gain or loss of electrons from migrating samples as they pass between electrodes at a given difference in electrical potential.

Mass spectrometry is an analytical technique used to measure the mass-to-charge ratio (m/z (or m/q)) of ions. It is most generally used to analyze the composition of a physical sample by generating a mass spectrum representing the masses of sample components. The technique has several applications, including: identifying unknown compounds by the mass of the compound and/or fragments thereof; determining the isotopic composition of one or more elements in a compound; determining the structure of compounds by observing the fragmentation of the compound; quantitating the amount of a compound in a sample using carefully designed methods (mass spectrometry is not inherently quantitative); studying the fundamentals of gas phase ion chemistry (the chemistry of ions and neutrals in vacuum); determining other physical, chemical or even biological properties of compounds with a variety of other approaches.

A mass spectrometer is a device used for mass spectrometry, and produces a mass spectrum of a sample to analyze its composition. This is normally achieved by ionizing the sample components and separating ions of differing masses (m/z ratios) and recording their relative abundance by measuring intensities of ion flux. A typical mass spectrometer comprises three parts: an ion source, a mass analyzer, and a detector.

The kind of ion source is a contributing factor that strongly influences what types of samples can be analyzed by mass spectrometry. Electron ionization and chemical ionization are used for gases and vapors. In chemical ionization sources, the analyte is ionized by chemical ion-molecule reactions during collisions in the source. Two techniques often used with liquid and solid biological samples include electrospray ionization (ESI) and matrix-assisted laser desorption/ionization (MALDI). Other techniques include fast atom bombardment (FAB), thermospray, atmospheric pressure chemical ionization (APCI), secondary ion mass spectrometry (SIMS) and thermal ionization.

In a preferred embodiment the detecting of an analyte in a method according to the present invention is performed by mass spectroscopy.

Nuclear magnetic resonance (NMR) detection is based on the fact that certain nuclei with odd-numbered masses, including H and ¹³C, spin about an axis in a random fashion. However, when placed between poles of a strong magnet, the spins are aligned either parallel or anti-parallel to the magnetic field, with the parallel orientation favored since it is slightly lower in energy. The nuclei are then irradiated with electromagnetic radiation which is absorbed and places the parallel nuclei into a higher energy state; consequently, they are now in "resonance" with the radiation. Each H or C will produce different spectra depending on their location and adjacent atoms or atom groups in the compound, because all nuclei in molecules are surrounded by electron clouds which change the encompassing magnetic field and thereby alter the absorption frequency.

When a source emits a parallel beam of light which strikes particles in solution, some light is reflected, absorbed, transmitted, or scattered. These phenomena can be measured by a light-scattering (LS) detector. The most prominent forms of LS detection are termed nephelometry and turbidimetry. Nephelometry is defined as the measurement of light scattered by a particulate solution. This method enables the detection of the portion of light scattered at a multitude of angles. Turbidimetry is defined as the measure of the reduction of light transmitted due to particles in solution. It measures the light scatter as a decrease in the light that is transmitted through the particulate solution. Therefore, it quantifies the residual light transmitted.

Near-infrared detectors operate by scanning compounds in a spectrum from 700 to 1100 nm. Stretching and bending vibrations of particular chemical bonds in each molecule are detected at certain wavelengths.

The method described herein can be used in the detection of an analyte from a sample of hemolyzed whole blood. As obvious to the skilled artisan the method according to the present invention will also work with other basic sample materials like plasma, serum, urine or CSF. A preferred sample to be used in a method according to the present invention thus can be selected from the group consisting of plasma, serum, urine, CSF and hemolyzed whole blood. A sample of hemolyzed whole blood is preferred.

As mentioned above, the authors of the present invention have also discovered a method for differential hemolysis. This method is not yet available to the public and therefore shall be described in some detail below. The term differential hemolysis relates to method of treating a sample comprising erythrocytes with a membrane solubilizing agent under conditions appropriate to lyse cell membranes of red blood cells and at the same time not to cause precipitation of sample constituents. As will be obvious from the below description, the method according to the present invention is preferably practiced with a sample of differentially hemolyzed whole blood.

"Red blood cells" in the sense of the present invention are red blood cells not having a cell nucleus. Such red blood cells not having a cell nucleus are e.g. the mature red blood cells as found in the circulation of mammals. This invention does not relate to nucleated red blood cells as e.g. known from avian species. The latter ones would meet the criteria for nucleated or eukaryotic cell.

"Mammal" for purpose of the present invention refers to any animal classified as a mammal, including humans, domestic and farm animals, and zoo, sports, or pet animals, such as dogs, cats, cattle, horses, sheep, pigs, goats, rabbits, etc. Preferably, the mammal is human.

A "eukaryotic cell" or "nucleated cell" in the sense of the present invention is a cell derived from a eukaryotic organism and still having its cell nucleus. Examples of eukaryotic cells are cells derived from nucleated tissue, nucleated tissue culture cells and nucleated blood cells. In a preferred embodiment the eukaryotic cell is a nucleated blood cell like a thrombocyte, a monocyte, neutrophils, eosinophils or a leukocyte. Cells from lower organisms, like bacteria, though containing genetic material, are not eukaryotic cells.

Hemolysis of a whole blood sample can be performed by any hemolysis method known in the art. Preferably the hemolyzed whole blood sample is obtained by differential hemolysis. The advantageous properties of differential hemolysis, i.e. of processing a liquid sample with a membrane solubilizing agent under conditions appropriate to lyse cell membranes of red blood cells and at the same time not to cause precipitation of sample constituents, as demonstrated in the Examples section, have been established by using whole blood samples.

Preferably the liquid sample subjected to a differential hemolysis with an appropriate membrane solubilizing agent comprises red blood cells and may comprise or comprises, respectively, nucleated cells. Further preferred the liquid sample comprises both red blood cells and nucleated cells. Preferably the liquid sample used in a method subjected to differential hemolysis and thereafter used in a method according to the present invention will be whole blood. As will be appreciated a whole blood sample contains both red blood cells without nuclei as well as nucleated blood cells.

Preferably the whole blood sample is processed directly, i.e. directly after sampling it is subjected to the differential hemolysis. Also preferred, the whole blood will be collected/treated with an appropriate anti-coagulant to yield an anti-coagulated whole blood sample. Well-known anti-coagulants frequently used in clinical diagnostic routine are heparin, citrate and EDTA. Preferably the sample according to the present invention is an anti-coagulated whole blood sample, especially a citrated whole blood sample or an EDTA-anti-coagulated whole blood sample that has been differentially hemolyzed.

In a method for differential hemolysis the liquid sample is treated in such a manner that two requirements are met: a) if red blood cells are present, the membranes of red blood cells are disrupted and b) at the same time no precipitation of sample constituents is caused. As mentioned, this process is termed differential hemolysis.

In case the method is practiced on a whole blood sample a processed sample, a differentially hemolyzed whole blood sample, is obtained containing lyzed red blood cells but at the same time no precipitate.

By applying a suitable membrane solubilizing agent under appropriate conditions the integrity of the cellular membrane that is e.g. essential for shielding the contents of a red blood cell from the blood plasma is lost. The content of the erythrocytes (e.g. hemoglobin but also some analytes of interest) is released into the surrounding liquid. At the same time no precipitation of sample constituents is caused.

As the skilled artisan will appreciate sample constituents that might interfere with a later analysis may especially be DNA and proteins. As long as the nuclei of eukaryotic cells, e.g. like lymphocytes or monocytes are not destroyed, no DNA is released from these nuclei. As long as no proteins precipitate, proteins comprised in the sample subjected to differential hemolysis will not interfere, at least not to a significant extend with a routine chromatography step. However, as illustrated further above, RBC constituents - especially hemoglobin - may nonetheless interfere with the detection of an analyte of interest.

The integrity of red blood cells can for example be easily assessed by appropriate life stains. In a preferred embodiment according to the present invention trypane blue is used in order to assess the integrity of a red blood cell membrane. Intact red blood cells do not accumulate trypane blue, whereas a red blood cell with a disrupted membrane does stain with trypane blue. The membrane integrity of a red blood cell is easily assessed under the microscope after staining a sample with trypane blue. The percentage of disrupted red blood cells is calculated by counting intact red blood cells before and after the treatment, by then dividing the first number by the latter number and by then multiplying this value by 100. Red blood cells that are solubilized are referred to as lyzed red blood cells or as lyzed erythrocytes.

The appropriate treatment will be adequate to lyse a red blood cell, but at the same time it will not cause precipitation of sample constituents. It is expected that the appropriate hemolysis treatment in a method according to the present invention will also effects the outer membranes of eukaryotic cells. However, care can and must be taken that the DNA contained in the cell nuclei is not released into the sample. The hemolysis reagent and conditions used will either and preferably leave the nuclear membrane and thus the nuclei macroscopically intact or at least DNA will not be set free from its surrounding and DNA-stabilizing nuclear proteins. If DNA would be released to a significant extend such DNA might or even would interfere with further handling of the sample. Released DNA e.g. tends to make the liquid very viscous. It is then no longer possible to pipette or transfer such sample or to pass it through certain filters or columns.

Care can and must also be taken that no protein precipitation occurs. As the skilled artisan will appreciate, there are many many different proteins present in a biological sample, e.g. in a whole blood sample. All these proteins have individual properties influencing their tendency to precipitate or aggregate.

It has now been found that it is possible to describe and define whether sample processing with a membrane solubilizing agent is performed under appropriate conditions i.e. in a mode to lyse cell membranes of red blood cells on the one hand and at the same time not to cause precipitation of sample constituents. Both, red blood cells not lysed as well as precipitated sample constituents would have a negative impact on the properties of such sample.

Whether the conditions for differential hemolysis are appropriate can be easily and preferably determined by using the following standardized procedure. A whole blood sample with a hematocrit of 40 is diluted 1:10 with the candidate hemolysis reagent. The efficacy of a reagent for bringing about differential hemolysis is seen visually. Upon lysis of the erythrocytes the mixture becomes clear. If precipitation of sample constituents occurs the sample becomes turbid or viscous or both.

As indicated above, the conditions used in a method of differential hemolysis according to the present invention can easily be assessed visually. If a whole blood sample is incubated with an appropriate candidate reagent for differential hemolysis the minimal concentration required to hemolyse red blood cells can be recognized as the lowest concentration rendering the turbid blood sample transparent or clear. The highest possible concentration is the one still leading to a transparent and non-viscous sample.

It has turned out rather easy to determine the appropriate minimal final concentration of the candidate hemolysis reagent as the concentration leading to the change in transparency of a treated whole blood sample. This change in transparency correlates well with the suitability of such processed sample for direct analysis by HPLC. However, for the sake of an unambiguous definition it is preferred that minimal concentration of a hemolysis reagent is confirmed by the HPLC method as described below.

The maximal concentration of hemolysis reagent possible is the concentration still not causing release of DNA and/or precipitation of a protein. The sample thereby would turn viscous or turbid or both and is not suitable for a direct HPLC application anymore. Whereas viscosity and turbidity can be followed visually it is preferred that maximal concentration of a hemolysis reagent is confirmed by an HPLC method as described below.

Both, a whole blood sample still comprising too many non-lysed erythrocytes as well as a treated whole blood sample comprising precipitated sample constituents will not be suitable for any chromatographic procedure. This is why the conditions appropriate to bring about differential hemolysis preferably are determined by applying in a standardized manner a sample of whole blood treated with a candidate reagent for differential hemolysis to an HPLC column.

Incomplete hemolysis and/or precipitation of sample constituents is assessed by applying 50 times 10 µl of the processed whole blood sample to an HPLC column. To assess whether a candidate hemolysis reagent for differential hemolysis is appropriate, said hemolysis reagent is mixed with a sample of whole blood. Preferably EDTA-blood with a hematocrit of 40 that has been prediluted 1:10 in physiological saline is used. It is mixed in a 1:1 ratio with the candidate hemolysis reagent and the mixture is incubated for 30 min at 20°C. 50 aliquots of 10 µL of the this mixture, i.e. a processed whole blood sample are applied to a filter with a diameter of 2 mm and 0.5 µm pore size that is part of an HPLC system. In case the frit is part of an HPLC column the stationary phase must be selected not to cause any interference or blocking. The back-pressure is monitored. A candidate reagent for differential hemolysis that would cause an increase in back-pressure of 20 bar or more - if the back-pressure for injection 50 and the back-pressure for the first injection are compared to each other - would be deemed not to be appropriate. This way both the minimal as well as the maximal final concentration of an appropriate reagent for differential hemolysis can easily be identified.

Preferably the filter used in the above assessment of a candidate reagent for differential hemolysis is an HPLC frit. Also preferred the frit is part of an HPLC column of 20 mm in length filled with 3.5 µm Symmetry^{®} C18 particles with a pore size of 100A° as bed material, and having an inner column diameter of 2 mm.

As the skilled artisan will readily appreciate the whole blood sample used for such assessment is obtained from a healthy individual, i.e. an individual having no known disease and biochemical values in the normal range.

It has been found and established that appropriate conditions can be established for quite many chemicals in order to meet both the requirements for differential hemolysis. The reagent for differential hemolysis comprises a membranolytic (= membrane solubilizing) chemical in the appropriate concentration to after mixing with the sample result in differential hemolysis.

The membrane solubilizing agent according to the present invention preferably is based on water as a solvent comprises a chemical or reagent bringing about the differential hemolysis as described above, and also preferred it may comprise a buffer and/or a preservative. The agents used for differential hemolysis preferably are based on chemicals or reagents with membrane solubilizing activity that have a molecular weight of less than 1000 Dalton.

The membrane solubilizing agent preferably is based on the membranolytic action of one or more of the following chemicals: KBr; KJ; and KSCN or on a salt consisting of one or more of the following cations and anions:

The cation preferably is selected from 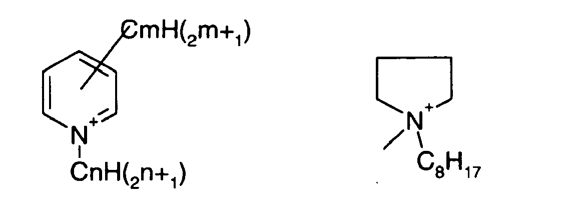 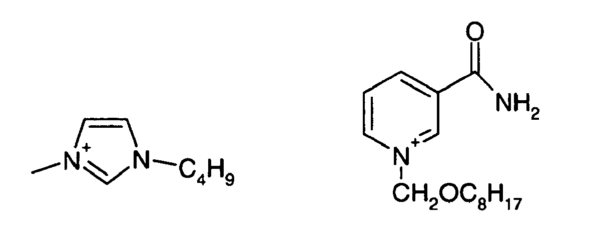 wherein m is 0 or 1 and n is 4 or 6.

The anion is preferably selected from chloride, tetrafluoroborate, octylsulfate, iodide und thiocyanate. It is also possible to use mixtures of the above mentioned chemicals. As the skilled artisan appreciates it is these chemicals that facilitate the differential hemolysis whereas other ingredients of a hemolysis reagent may serve different purposes and may e.g. function as a buffer or as a preservative.

Preferably the chemical comprised in a reagent for differential hemolysis is a salt wherein the cation preferably is selected from 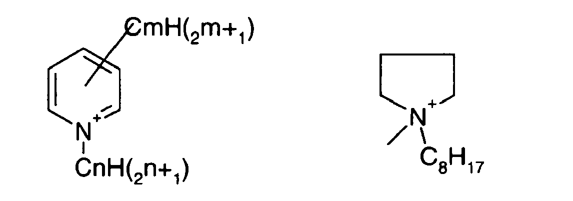 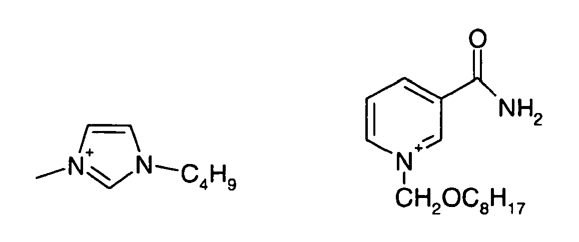 wherein m is 0 or 1 and n is 4 or 6, and wherein the anion is preferably selected from chloride, tetrafluoroborate, octylsulfate, iodide und thiocyanate.

Appropriate membranolytic chemicals comprised in a membrane solubilizing agent are preferably selected from the group consisting of 1-Butyl-4-methylpyridinium tetrafluoroborate; 1-Butyl-3-methyl-imidazolium tetrafluoroborate; 1-Butyl-3-methyl-imidazoliumoctylsulfate; 1-Butyl-3-methyl pyridiniumchloride; 1-Hexylpyridiniumchloride; 1-Methyl-1-octyl pyrrolidiniumchloride; N-Octylpyridiniumchloride; 3-Carbamoyl-1-octyloxymethyl pyridiniumchloride; KBr; KJ; and KSCN, and of combinations thereof.

Preferably the membranolytic chemicals comprised in a membrane solubilizing agent are selected from the group consisting of 1-Butyl-4-methylpyridinium tetrafluoroborate; 1-Butyl-3-methyl-imidazolium tetrafluoroborate; 1-Butyl-3-methyl-imidazoliumoctylsulfate; 1-Butyl-3-methyl pyridiniumchloride; 1-Hexylpyridiniumchloride; 1-Methyl-1-octyl pyrrolidiniumchloride; N-Octylpyridiniumchloride; and 3-Carbamoyl-1-octyloxymethyl pyridiniumchloride. It is further preferred to use a mixture of one these reagents and of KSCN.

As obvious to the skilled artisan, once an appropriate concentration of a candidate reagent for differential hemolysis has been identified in the above defined method that is based on a 1 in 20 dilution of a whole blood sample in a candidate hemolysis reagent, another ratio of whole blood sample to an adjusted hemolysis reagent can be used as required.

In case the analyte of interest is expected to be highly concentrated in the blood sample under investigation, the final concentration of the hemolysis reagent can stay the same as identified in the above setting and lower ratios of whole blood to hemolysis reagent, e.g., 1:30, 1:40 or 1:50 can be used. Preferably in a membrane solubilizing agent according to the present invention the reagent for differential hemolysis is used in at least the minimal concentration sufficient to achieve differential hemolysis as determined above.

In case the analyte of interest is present in rather a low concentration it may be necessary not to dilute the whole blood sample 1:20 but less. This is feasible by adjusting the concentration of the hemolysis reagent accordingly, such that the final relative concentration of hemolysis reagent to whole blood in the mixture of the hemolysis reagent and the whole blood sample stays within the ratio identified for the required minimal and maximal concentration, respectively, of hemolysis reagent as determined in the above described assessment.

By way of example: It has been found that 1-Methyl-1-octyl pyrrolidiniumchloride/KSCN if used in a final concentration of 1% and 0.4% in a membrane solubilizing agent according to the present invention, respectively, are appropriate to achieve the desired result, i.e. the differential hemolysis of a whole blood sample at a final dilution of 1:20. Dilution of an analyte in the processed blood sample can be reduced if for example the concentration of this hemolysis reagent is adjusted to 2% for 1-Methyl-1-octyl pyrrolidiniumchloride and 0.8% for KSCN, respectively. The membrane solubilizing agent comprising this adjusted concentration of hemolysis reagent, if later mixed 1:1 with a 1:5 diluted whole blood sample, also leads to differential hemolysis of the whole blood sample. Since the ratio of whole blood to hemolysis reagent is kept constant, this processed blood sample is only diluted 1:10. If 1 ml of a membrane solubilizing agent comprising 10% of 1-Methyl-1-octyl pyrrolidiniumchloride and 4% of KSCN, respectively, is mixed with 1 ml of whole blood diluted 1:1 in PBS differential hemolysis is also observed. Alternatively 1 ml of whole blood could be added to 2 ml of a membrane solubilizing agent comprising 10% of 1-Methyl-1-octyl pyrrolidiniumchloride and 4% of KSCN, respectively.

For many routine applications it is expected that the ideal ratio of whole blood sample to a membrane solubilizing agent will be between 10:1 and 1:20. Preferably in a method according to the present invention the sample of whole blood is mixed with the hemolysis reagent at a ratio from 5 to 1 to 1 to 15. More preferred the ratio is between 2 to 1 and 1 to 10, also preferred between 1 to 1 and 1 to 5. The final, i.e. highest possible concentration of an adjusted hemolysis reagent used in the clinical routine will depend on the solubility and also the price of such reagent.

Preferably an appropriate reagent for different hemolysis is further characterized in that the (minimal) concentration of the chemical required for disrupting the membrane of a red blood cell and the (maximal) concentration tolerated for said chemical at which at the same time no precipitation of sample constituents is caused are at least two-fold apart. The broader the window between minimal and maximal concentration for a the reagent responsibly for differential hemolysis the more easily such reagent can be used in clinical diagnostic routine.

It is further preferred that the membranolytic chemical comprised in a reagent for differential hemolysis is used at a concentration that after mixing said reagent with a sample the final concentration of this membranolytic chemical corresponds to the mean value plus 30% of the minimal concentration minus 30% the maximal concentration, respectively. Further preferred the concentration of the membranolytic chemical comprised in the reagent for differential hemolysis will be adjusted that after mixing it with a sample it is within plus or minus 25%, 20% or 15% of the mean value of the minimal and maximal concentration, respectively.

It is also preferred that the membranolytic chemical comprised in a reagent for differential hemolysis is used at a concentration that after mixing with a sample a final concentration of this hemolytic chemical corresponding to a concentration between one and four times the minimal concentration, and also preferred between 1.5 times and 3 times the minimal concentration determined as described above is obtained.

Preferably the reagent for differential hemolysis in a membrane solubilizing agent of the present invention is used at a concentration of no more than 75% weight/volume, also preferred at no more than 50% weight/volume.

Preferably the method of differentially hemolyzing a sample is combined with a method according to the present invention. Preferably this method also uses a further liquid chromatography (LC) step.

In a further preferred embodiment the present invention relates to a method of detecting an analyte in a liquid sample the method comprising the steps of obtaining said liquid sample, subjecting said liquid sample to a method of sample processing by a membrane solubilizing agent, wherein the solubilizing agent is appropriate to disrupt the membrane of red blood cells, and not to destroy the nuclei of eukaryotic cells, if required, adjusting the pH to a value above pH 8.0, applying the sample to a RAM, and thereafter eluting the bound material, subjecting it to liquid chromatography and analyzing the analyte under investigation by appropriate means. Preferably the steps of applying the sample to RAM, eluting and chromatographing the sample as well as the method of analysis are all performed directly (online).

In routine applications the stationary phase, the so-called bed material, e.g. derivatized silica particles in an RP-HPLC-application, is packed into an appropriate column, and is protected by a frit. The frit material usually is selected to have e.g. a smaller pore size as compared to the pore size of the bed material.

In HPLC methods the diameter of the stationary phase particles usually is in the range of 1 to 10 µm. These small particles necessitate the high pressure used in HPLC. The bed material usually is protected by a frit. Typical frits have a pore size of 1 µm, 0.45 µm or 0.2 µm. The smaller the particles the smaller is usually the pore size of the frit. If a sample comprises a constituent capable of blocking an HPLC frit this is detrimental for any routine analysis. A whole blood sample, as well as an "over-treated" whole blood sample comprising precipitates of sample constituents causes a rapid blocking of any routine HPLC frit or column. As the skilled artisan will appreciate blocking of the frit used in an HPLC column will occur the more rapidly the lower the pore size of the frit, the smaller the diameter of the stationary phase particles and the smaller the column diameter. In case the frit would not be selected appropriately, i.e. a too large pore size, the particle size of the column material would also matter and the column itself would block more rapidly the smaller the particles are.

By subjecting a sample to differential hemolysis, e.g. to a sample of whole blood, it is possible to directly apply such treated sample to an HPLC column, without running the risk of blocking the column. Preferably, the stationary phase particles used in such HPLC step are in the range of 1 to 10 µm, also preferred in the range of 2 to 7 µm in diameter. Preferably the frit used in such HPLC step has a pore size of 0.5 µm or also preferred of 0.2 µm.

As illustrated in the Examples, it is now possible to subject a sample, e.g. of whole blood to differential hemolysis, to apply such hemolysate to a RAM under conditions wherein specific pH requirements are met, to elute the RAM-bound material and to detect the absence, presence or quantity of an analyte of interest like an antibiotic or an immunosuppressive drug, like tacrolimus or rapamycin.

### Description of the Figures

- Figure 1: pH 6.6: Chromatogram of hemoglobin at 396 nm. Peak #1 represents hemoglobin in the flow through, peak #2 represents hemoglobin as eluted at 15% buffer B and peak #3 represents hemoglobin found once the column is washed with 100% buffer B, respectively.
- Figure 2: pH 10.7: Chromatogram of hemoglobin at 396 nm. Peak #1 represents hemoglobin in the flow through; peak #2 represents hemoglobin as eluted at 20% buffer (B), respectively.
- Figure 3: Investigation of hemoglobin carry-over at pH 10.7. Hemoglobin carry-over was investigated by performing a blank injection after the analysis of a haemoglobin-containing sample. As can be seen from the chromatogram, no haemoglobin can be identified at 20% buffer (B).
- Figure: Experimental setup used to extract analytes from hemolysates. Hemoglobin was monitored after Biotrap 500 MS with UV-Vis detection. Analytes were detected after the analytical column by mass spectrometry.
- Figure 5: Blank injection of whole blood hemolysate. The chromatogram shows that at the position where tetracycline should elute, no peak is found.
- Figure 6: Injection of whole blood hemolysate spiked with tetracycline. The chromatogram shows that at the position where tetracycline should elute, a clear peak for this analyte is found.
- Figure 7: Blank chromatographic run on Biotrap 500 MS after an injection of hemolysate as a sample. As obvious from this chromatogram no hemoglobin is detected.
- Figure 8: Calibration curve for tetracycline spiked into a human whole blood hemolysate. A linear correlation is observed as indicated by the extrapolation line shown in this Figure and by the high coefficient of correlation calculated (r² = 0.945).
- Figure 9: Analytical HPLC setup used to extract and detect immuno suppressive drugs from a whole blood hemolysate.
- Figure 10: Extraction of tacrolimus from a whole blood hemolysate with on- line SPE-HPLC-UV. The chromatogram taken at 291 nm (the maximal adsorption of tacrolimus) is depicted. The arrow indicates the peak corresponding to tacrolimus.
- Figure 11: Extraction of rapamycin from a whole blood hemolysate with on- line SPE-HPLC-UV. The chromatogram taken at 278 nm (the maximal adsorption of rapamycin) is depicted. The arrow indicates the peak corresponding to rapamycin.

### Example 1

### Evaluation of various RAMs at different pH values

The RAM columns were directly installed into an analytical HPLC system. Ten µL of hemoglobin solution were injected. First tests were performed with sample application buffers (Eluent buffer A) of different pH of 6.6 and at pH 10.7, respectively. For each pH value, amounts of hemoglobin flowing through and retained on the column were computed. Carry-overs were also evaluated by performing blank injections consecutive to injections of hemoglobin.

The column effluent was monitored at 396 nm (the latter being the absorption maximum of hemoglobin). The columns tested included LiChrospher^{®} RP-18 ADS from Merck (Merck, Darmstadt, Germany, ordering number 1.50947.0001), and Biotrap 500 MS from ChromTech Ltd., Cogleton, United Kingdom, ordering number BMS134K

### a) Evaluation of hemoglobin interference at pH 6.6 (5mM ammonium acetate)

A LiChrospher^{®} RP-18 ADS column from Merck was used. 10 µL of sample (150 mg/mL of hemoglobin dissolved in 5 mM ammonium acetate at pH 6.6) was applied to the column. For elution of hemoglobin a mobile phase using the two buffers (A) 5 mM ammonium acetate in H₂O (pH 6.6) and (B) 5 mM ammonium acetate in acetonitrile (ACN) (pH 6.6), respectively was used. Elution was performed for 15.0 min isocratic at 100 % (A), then using a linear gradient from 100% (A)-0% (B) to 0% (A)-100% (B) in 30.0 min, and thereafter by a wash step for 2.0 min at 100 % (B) to detect any hemoglobin reversibly bound to the RAM. The flow rate was 1.5 mL/min and all steps were carried out at room temperature.

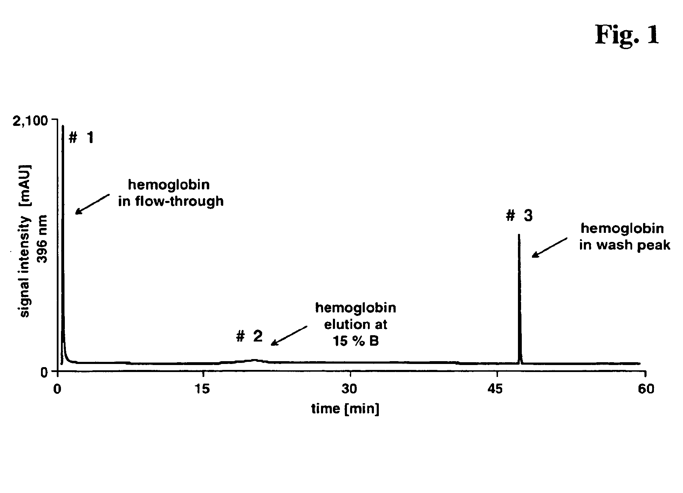

At pH 6.6, hemoglobin is predominantly present in the flow-through (set to 100 %), partially elutes at around 15 % B (24 %), and is also present in the wash step at 100 % (B) (4 %). However, this separation is not always reproducible and the correct quantitation of both the unretained as well as the retained hemoglobin, respectively, is rather difficult because of detector saturation with the flow-through peak and the large width and the flatness of the retained peaks (cf. Figure 1). Hemoglobin in the wash peak is critical, since this would most likely imply interference by hemoglobin in an online set-up for an analyte measurement from a hemolysate. Since hemoglobin was already present in the wash step, the risk of hemoglobin carry-over, i.e. the risk of interference in any further analyte detection by hemoglobin contained in a sample previously applied to the online system is very high and would for example result in contamination of electrospray ion sources used for mass spectrometric detection.

### b) Evaluation of hemoglobin interference at pH 10.7 (10 mM ethanolamine)

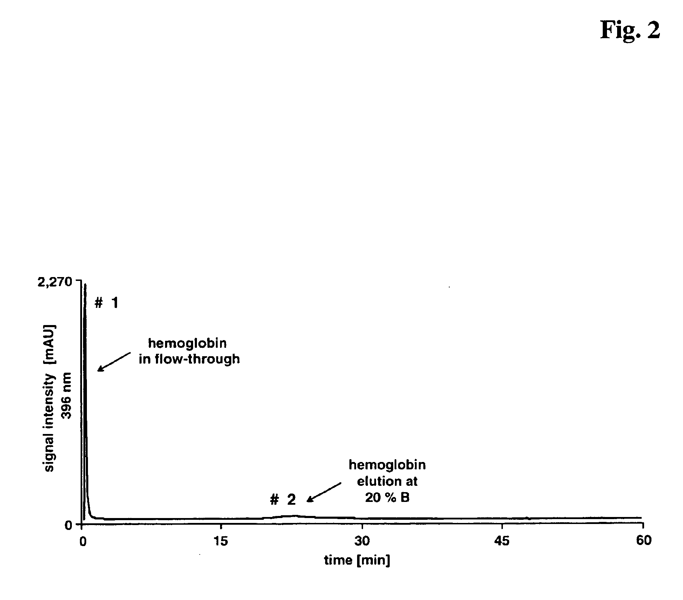

A LiChrospher^{®} RP-18 ADS column from Merck was used. 10 µL of sample (150 mg/mL of hemoglobin dissolved in 10 mM ethanolamine at pH 10.7) was applied to the column. For elution of RAM-bound hemoglobin a mobile phase using the two buffers (A) 10 mM ethanolamine in H₂O (pH 10.7) and (B) 10 mM ethanolamine in ACN (pH 10.7), respectively was used. Elution was performed for 15.0 min isocratic at 100 % (A), then using a linear gradient from 100% (A)- 0% (B) to 0% (A)-100% (B) in 30.0 min, and thereafter by a wash step for 2.0 min at 100 % B. The flow rate was 1.5 mL/min and all steps were carried out at room temperature. At pH 10.7, hemoglobin is predominantly present in the flow-through (set to 100 %), partially elutes at around 20 % (B) (20 %), and is not present to any significant level in the cleaning peak (cf. Figure 2). The absence of hemoglobin from the cleaning peak as observed after application of 100 % (B) is very important, since this would indicate that under these buffer conditions hemoglobin would not cause any memory effect, i.e. it would not be present in the next measurement using the same column. Practically no carry-over was observed at pH 10.7. This pH value appears to be appropriate to avoid carry-over of hemoglobin between different injections onto RAM and to thus to avoid memory effects.

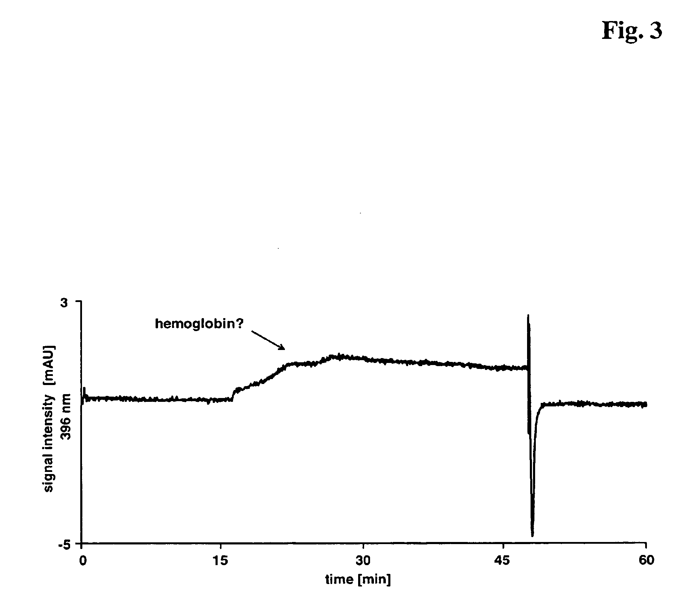

In order for an online method to be fully compatible with routine requirements, such method also necessitates not be hampered by carry-over problems. When measuring an analyte from a sample of hemolyzed whole blood, carry-over problems would most likely result from hemoglobin carry-over, since hemoglobin represents the by far most abundant polypeptide in a hemolysate. Hemoglobin carry-over was investigated by performing a blank injection after the hemoglobin sample. Instead of a sample containing hemoglobin 10 µL of 10 mM ethanolamine (pH 10.7) were applied to the same column as above. All other buffer/elution conditions remained unchanged. As can be seen from Figure 3, no hemoglobin can be identified in this blank injection. This means under this buffer conditions hemoglobin is neither found in the flow-through nor in the next run. Under these buffer conditions no hemoglobin would interfere with measurement of an analyte of interest.

It should be mentioned that these very positive observations were made under buffer/run conditions that are far above the pH-range as specified by the manufacturer for this RAM column.

### Example 2

### Quantitation of tetracycline hydrochloride in human whole blood hemolysates

In this series of experiments a Biotrap 500 MS^{®} column has been used. Whole blood has been differentially hemolyzed and used as a sample or as a matrix for spiking with an analyte of potential interest. Due to the positive experiences described in Example 1, application and elution buffers with a pH of 10.7 were used. For specific analyte detection a linear quadrupole mass spectrometer was used.

### a) Preparation of blood hemolysates

Anti-coagulated whole blood was stored at +4°C until analysis. The reagent for differential hemolysis was prepared by mixing 0.7 g C₁₃H₂₈CIN (1-methyl-octyl-pyrrolidinium chloride) and 0.3 g KSCN in 25 mL H₂O.

Blood hemolysates were obtained by mixing under gentle agitation a sample of 1:10 diluted blood (diluted in 150mM NaCl) and lysis reagent in a 1 to 1 ratio. After a few minutes the blood sample became clear and ready for injection in the HPLC-MS system. Unfortunately the striking effect of differential hemolysis can not be easily visualized on black and white reproductions. Therefore no picture is shown. However, this procedure, resulting in a clear sample of differentially hemolyzed whole blood, can easily be reproduced by the skilled artisan. It should be mentioned that preparation of appropriate (differential) hemolysates was not possible after dilution of blood in 10 mM ethanolamine. Consequently, blood hemolysates were first prepared at neutral conditions by mixing 300 µL whole blood to 2.7 mL of 150 mM NaCl and then wit 3 mL lysis reagent. The sample of differentially hemolyzed blood was then injected under basic conditions (10 mM ethanolamine, pH 10.7) in the SPE-HPLC-MS setup as described below.

### b) Experimental setup

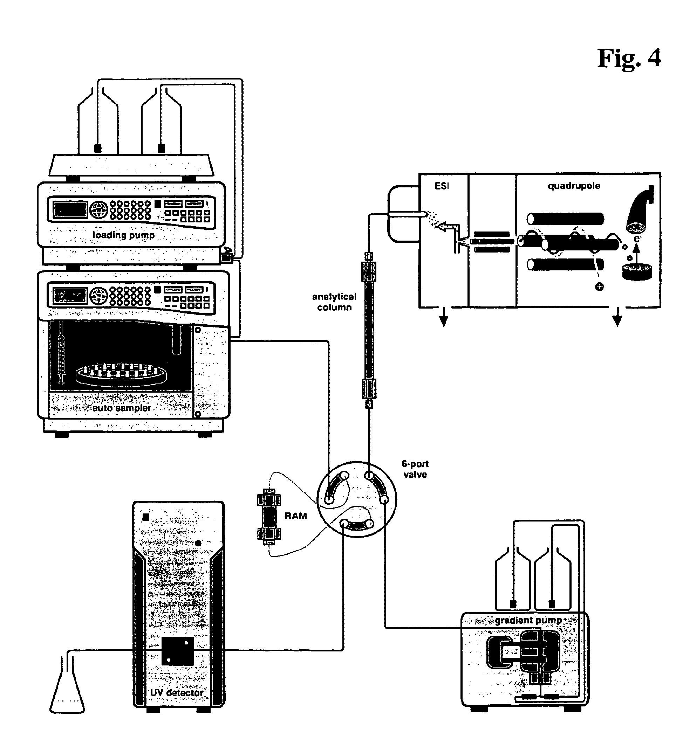

A scheme showing the analytical setup is depicted in Figure 4. The sample was injected over Biotrap 500 MS at pH 10.7 and after the hemoglobin comprised in the sample had flown through the column, the analyte of interest was transferred to HPLC and mass spectrometric detection by switching a 6-way valve. The loading pump was a P680 HPLC pump from Dionex, Germany, and the injection system consisted of a Rheodyne 6-port valve (7725) mounted with a 2.5 mL external loop. Hemoglobin monitoring was performed with a diode array detector UVD340U (Dionex) implemented after the Biotrap 500 MS trap column. The switching unit consisted of a Rheodyne 6-port valve (7000). The elution was performed over a Prontosil 300-5-C18-H 5 µm (125 x 2.0 mm) analytical column from Bischoff Analysentechnik und Geräte Leonberg, Germany. Eluent delivery was accomplished with a Rheos 2000 pump from Flux Instruments, Switzerland. MS detection of the analytes was performed with a linear quadrupole Surveyor MSQ from Thermo Finnigan, CA, USA.

### c) Detection of tetracycline

Two different samples were used and corresponding chromatograms compared to one another. The first sample was pure hemolysate to which no tetracycline had been added. The second one was hemolysate spiked with tetracycline. For the second sample 30 ng of tetracycline hydrochloride were spiked in 3 mL whole blood hemolysate (150 µL blood + 1,350 µL 150 mM NaCl + 1,500 µL of lysis reagent). The resulting concentration of tetracycline hydrochloride should correspond to 200 pg/µL in whole blood.

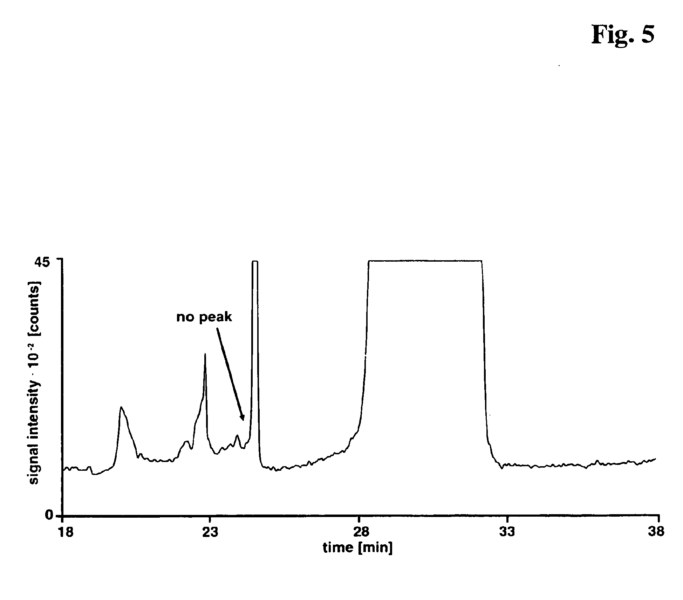

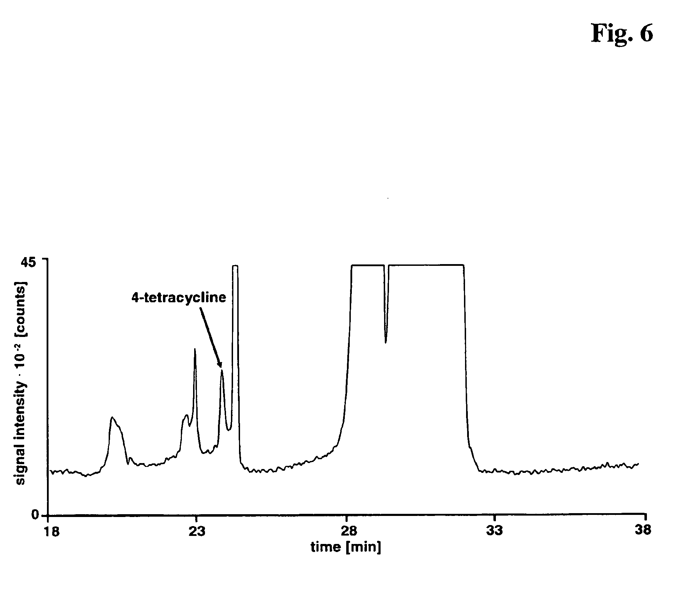

2.5 mL of each sample were injected over Biotrap 500 MS and washed with 10 mM ethanolamine at 3.2 mL/min. After 18 min the 6-port valve was switched and the elution occurred over the analytical column at 300 µL/min. (buffer (A): H₂O + 0.05 % TFA, buffer (B): ACN + 0.05 % TFA; linear gradient 10-30 % (B) in 7.5 min followed by 30-100 % (B) in 2.5 min and isocratic conditions of 100% (B) for 2 min). Mass spectrometric detection was performed by monitoring *m*/*z* 445.2 ± 1.0. Ion monitoring chromatograms corresponding to a blank run and to an injection of 200 pg tetracycline hydrochloride per µL of blood are depicted in Figures 5 and 6, respectively. As shown in these Figures, it was possible to detect tetracycline at 200 pg/µL in whole blood hemolysates. However, the neighboring elution of a compound with the same *m*/*z* does not permit to detect tetracycline at much lower levels or in less concentrated samples. This problem will be addressed in future experiments by using selected reaction monitoring on an ion-trap- or triple quadrupole mass spectrometer.

### d) Carry-over experiments

To check the carry-over of hemoglobin on Biotrap 500 MS, 2.5 mL of a whole blood hemolysate were injected. The loading step on Biotrap 500 MS was performed with 10 mM ethanolamine at 3.2 mL/min for 18 min. The 6-port valve was then switched and back flush elution was performed with a gradient of ACN + 0.05 % TFA (10-30 % (B) in 7.5 min, 30-100 % (B) in 2.5 min, 100 % (B) for 2.0 min, and 0 % (B) for 7.9 min).

During the back flush elution, the sample loop was washed by applying a gradient of 10 mM ethanolamine in H₂O (A) to 10 mM ethanolamine in ACN (B), (i.e., 0 % (B) for 2 min, 0-100 % (B) in 4 min, 100 % (B) for 6 min, and 0 % (B) for 7.9 min).

The carry-over of hemoglobin on Biotrap 500 MS was checked by performing a blank chromatographic run after the injection of hemolysate. Detection was performed at 396 nm.

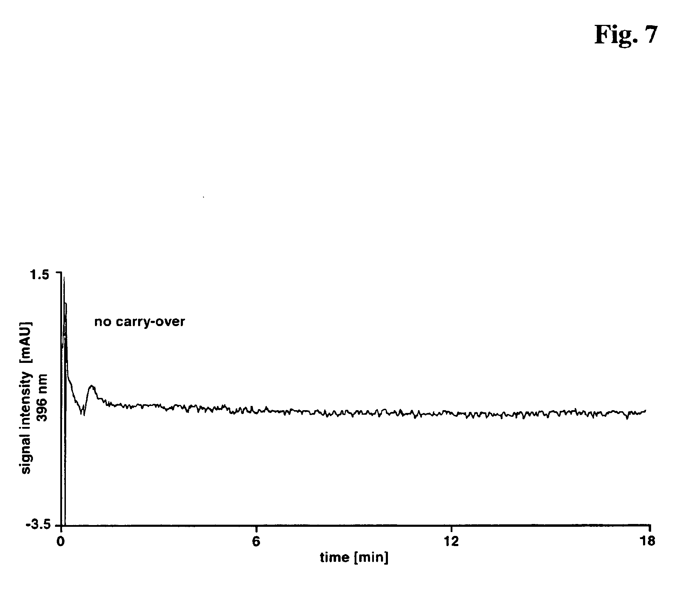

As obvious from Figure 7, no carry-over of hemoglobin was observed even when the hemolysate obtained by lyzing a sample of whole blood was applied as a sample on Biotrap 500 MS at pH 10.7.

### e) Calibration curve

Five samples consisting of tetracycline hydrochloride spiked into blood hemolysate were prepared. The concentrations of tetracycline hydrochloride in whole blood were 200 pg/µL, 600 pg/µL, 1.000 pg/µL, 1.500 pg/µL, and 2.000 pg/µL. The corresponding concentrations in the hemolysate solutions to be injected were: 9.5 pg/µL, 28.5 pg/µL, 47.5 pg/µL, 70.9 pg/µL, and 94.3 pg/µL. For each sample, 2.5 mL were injected and analyzed as described in (c) above. Samples were analyzed twice or three times.

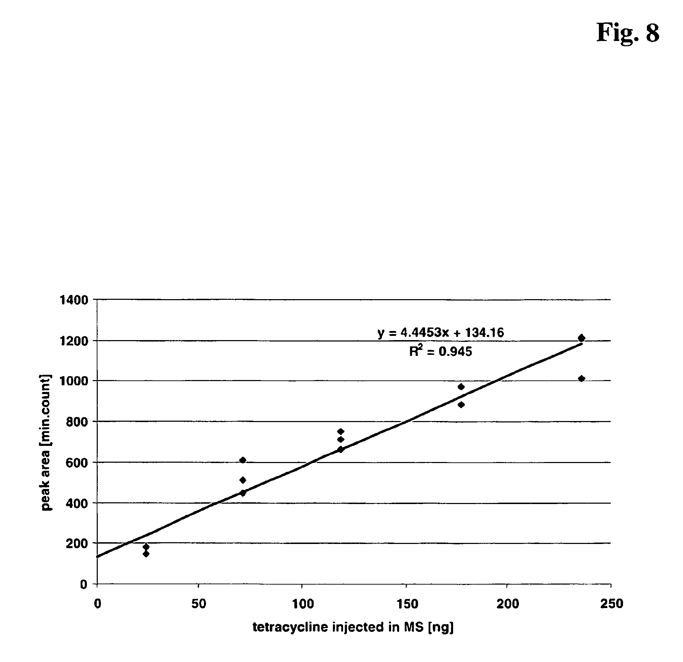

A calibration curve was computed and is plotted in Figure 8. A linear correlation is observed (R² = 0.945), proving the ability of Biotrap 500 MS to quantitatively extract tetracycline and thus most likely also other antibiotics from whole blood hemolysates at clinically meaningful concentrations (200 pg/µL in whole blood). MRM detections on triple quadrupole analyzers should permit to avoid integration errors due to coelution of the analyte with other compounds and thus lead to even further improved measurement of antibiotics. MRM detections are expected to permit that even lower limits of detection can be achieved.

### Example 3

### Extraction of immunosuppressive drugs from a whole blood hemolysate

In Example 2, the ability of the Biotrap 500 MS RAM to extract antibiotics from a whole blood hemolysate has been shown. Encouraged by these positive results further investigations were initiated whether the method might be applicable to other analytes of interest as well. Since immunosuppressive drugs are very important clinically relevant analytes, it was investigated whether this class of analytes might now be also amenable to an elegant online detection procedure based on the method described and used in Example 2.

The analytes investigated were tacrolimus and rapamycin. Tacrolimus is commercially available under FK-506 monohydrate and was purchased from Sigma and (CAS # 109581-93-3). Rapamycin (also called sirolimus) is also available from Sigma (CAS # 53123-88-9).

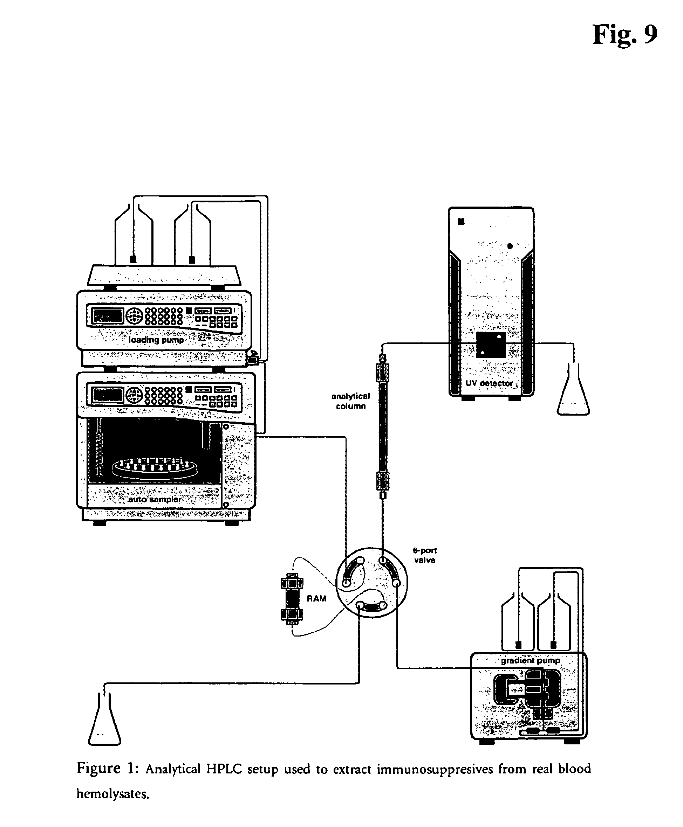

A whole blood hemolysate was spiked with different amounts of an immunosuppressive drug. The sample was injected and extracted over Biotrap 500 MS at pH 10.7. After 10 min, the immunosuppressive drug (i.e., the analyte of interest) was transferred to the analytical column by switching a 6-way valve. A scheme of the analytical setup is depicted in Figure 9. The loading pump was a P680 HPLC pump from Dionex and the injection system consisted of a Rheodyne 6-port valve (7725) mounted with a 2.5 mL external loop. The switching unit consisted of a Rheodyne 6-port valve (7000). The elution was performed over a Prontosil 300-5-C18-H 5 µm (125 x 2.0 mm) analytical column from Bischoff. Eluent delivery was accomplished with a Rheos 2000 pump from Flux Instruments. Analyte monitoring was performed with a diode array detector UVD340U (Dionex) implemented after the analytical column. Thus unlike to the setting of Example 2 e) here a less sensitive and selective means of detection was chosen.

A hemolysate without spiking of any immunosuppressive drug was first injected into the HPLC system and monitored at 291 nm and 278 nm (i.e. the maximal absorption of tacrolimus and rapamycin, respectively).

Then, solutions of tacrolimus and rapamycin were injected into the HPLC-UV in order to determine their retention time. Large amounts of immunosuppressive were injected into the HPLC-UV system in order to get unambiguous identifications.

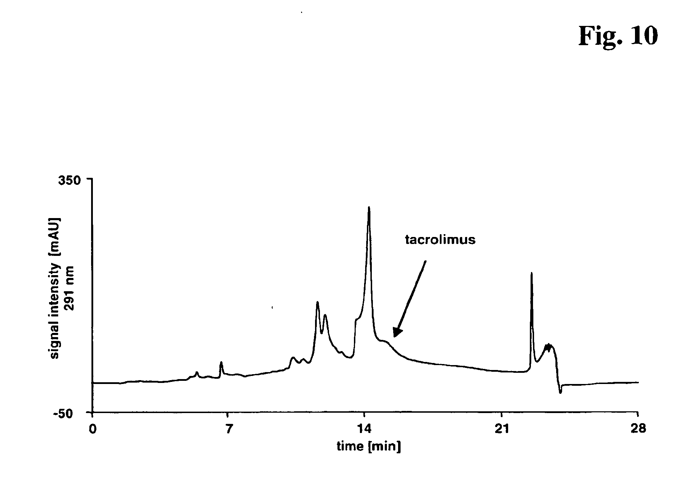

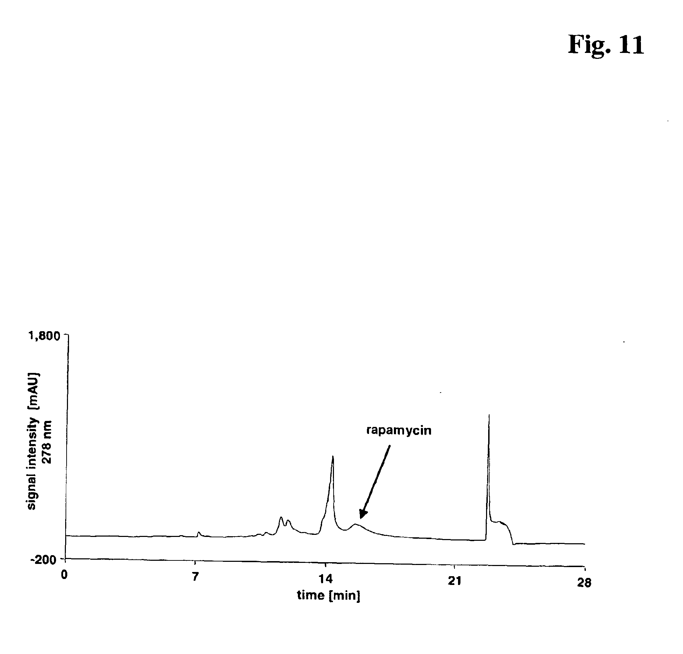

Finally, tacrolimus and rapamycin were spiked into whole blood hemolysates and analyzed with on-line SPE-HPLC-UV. Chromatograms are depicted in Figure 10 and in Figure 11, obtained after spiking tacrolimus and rapamycin into a hemolysate corresponding to a concentration of 291.1 ng/µL and was 20.0 ng/µL, respectively. As demonstrated (indicated by the arrow in these Figures), it was possible to unambiguously identify the tacrolimus and rapamycin spiked into a whole blood hemolysate. As the skilled artisan will note, the amounts of immunosuppressive drugs injected into the SPE-HPLC-UV system were rather high. The limits of detection using diode-array UV-detection are at about 291 ng/µL and at about 20 ng/µL for tacrolimus and rapamycin, respectively. However, as the skilled artisan will readily appreciate clinically relevant lower detection limits should be achieved when using for example selected reaction monitoring tandem mass spectrometry as described above for determination of tetracycline.

Therefore in conclusion it appears that by use of appropriate buffers in combination with commercially available RAM the online detection of many analytes of interest from a whole blood sample is now possible and may have significant clinical diagnostic as well as commercial utility.

## Claims

1. A method of detecting an analyte in a hemolyzed whole blood sample the method comprising the steps of
a) applying the sample of hemolyzed whole blood known or suspected to contain the analyte of interest to a column comprising a restricted access chromatography material (RAM) thereby binding the analyte,
b) eluting the analyte from the RAM and,
c) detecting the analyte,
wherein at least in step (a) a buffer with a pH above 8.0 is used.

2. The method of claim 1, wherein the RAM is selected from porous silica or porous polymer-based particles.

3. The method according to claim 1 or 2, wherein the inner surface of the RAM is binding the analyte of interest by hydrophobic interaction, ionic interaction or by polar interaction.

4. The method according to claim 3, wherein the porous silica or polymer-based particles have a hydrophobic inner surface of the pores and a hydrophilic coating outside of the pores.

5. The method according to any of claims 1 to 4, wherein the inner surface of the RAM is binding the analyte of interest by hydrophobic interaction.

6. The method according to any of claims 1 to 5, wherein the pore size of the RAM is between 60 and 120 Å.

7. The method according to any of claims 1 to 4, wherein the protein fraction includes polypeptides of 15 kDa and larger.

8. The method according to any of claims 1 to 7, wherein the analyte after elution from the RAM column is further separated by reversed phase HPLC.

9. The method according to any of claims 1 to 8, wherein the analyte has a molecular weight of 10 kDa or less.

10. The method according to any of claims 1 to 9, wherein the analyte is selected from the group consisting of immunosuppressive drugs, drugs of abuse, folic acid and folic acid vitamers.

## Patentansprüche

1. Verfahren zum Nachweis eines Analyten in einer hämolysierten Vollblutprobe, bei dem man
a) die Probe des hämolysierten Vollbluts, von dem bekannt ist oder vermutet wird, dass es den betreffenden Analyten enthält, auf eine Säule aufträgt, die ein chromatographisches Material mit eingeschränkter Zugänglichkeit (Restricted Access Material, RAM) umfasst und so den Analyten bindet,
b) den Analyten vom RAM eluiert und
c) den Analyten nachweist,
wobei man wenigstens in Schritt (a) einen Puffer mit einem pH-Wert von über 8,0 verwendet.

2. Verfahren nach Anspruch 1, wobei das RAM aus porösem Kieselgel und porösen Partikeln auf Polymerbasis ausgewählt ist.

3. Verfahren nach Anspruch 1 oder 2, wobei die innere Oberfläche des RAM den betreffenden Analyten durch hydrophobe Wechselwirkung, ionische Wechselwirkung oder polare Wechselwirkung bindet.

4. Verfahren nach Anspruch 3, wobei das poröse Kieselgel oder die Partikel auf Polymerbasis eine hydrophobe innere Oberfläche der Poren und eine hydrophile Beschichtung außerhalb der Poren aufweist/aufweisen.

5. Verfahren nach einem der Ansprüche 1 bis 4, wobei die innere Oberfläche des RAM den betreffenden Analyten durch hydrophobe Wechselwirkung bindet.

6. Verfahren nach einem der Ansprüche 1 bis 5, wobei die Porengröße des RAM zwischen 60 und 120 Å liegt.

7. Verfahren nach einem der Ansprüche 1 bis 4, wobei die Proteinfraktion Polypeptide mit einer Größe von 15 kDa und größer beinhaltet.

8. Verfahren nach einem der Ansprüche 1 bis 7, wobei der Analyt nach der Elution von der RAM-Säule weiter durch Umkehrphasen-HPLC aufgetrennt wird.

9. Verfahren nach einem der Ansprüche 1 bis 8, wobei der Analyt ein Molekulargewicht von 10 kDa oder weniger aufweist.

10. Verfahren nach einem der Ansprüche 1 bis 9, wobei der Analyt aus der aus Immunsuppressiva, Drogen, Folsäure und Folsäurevitameren bestehenden Gruppe ausgewählt ist.

## Revendications

1. Procédé de détection d'un analyte dans un échantillon de sang total hémolysé, le procédé comprenant les étapes consistant à :
a) déposer l'échantillon de sang total hémolysé contenant l'analyte ou censé contenir l'analyte présentant un intérêt sur une colonne comprenant un matériau pour chromatographie à accès restreint (RAM) se liant ainsi à l'analyte,
b) éluer l'analyte à partir du RAM, et
c) détecter l'analyte,
dans lequel au moins dans l'étape (a), on utilise un tampon ayant un pH supérieur à 8,0.

2. Procédé selon la revendication 1, dans lequel le RAM est choisi parmi une silice poreuse ou des particules polymères poreuses.

3. Procédé selon la revendication 1 ou 2, dans lequel la surface interne du RAM se lie à l'analyte présentant un intérêt par interaction hydrophobe, interaction ionique ou interaction polaire.

4. Procédé selon la revendication 3, dans lequel la silice poreuse ou les particules polymères poreuses a/ont une surface interne hydrophobe des pores et un revêtement hydrophile à l'extérieur des pores.

5. Procédé selon l'une quelconque des revendications 1 à 4, dans lequel la surface interne du RAM se lie à l'analyte présentant un intérêt par interaction hydrophobe.

6. Procédé selon l'une quelconque des revendications 1 à 5, dans lequel la taille des pores du RAM est comprise dans la plage allant de 60 à 120 Å.

7. Procédé selon l'une quelconque des revendications 1 à 4, dans lequel la fraction de protéine comprend des polypeptides de 15 kDa et plus.

8. Procédé selon l'une quelconque des revendications 1 à 7, dans lequel l'analyte après élution à partir de la colonne de RAM est en outre séparé par HPLC en phase inverse.

9. Procédé selon l'une quelconque des revendications 1 à 8, dans lequel l'analyte possède un poids moléculaire inférieur ou égal à 10 kDa.

10. Procédé selon l'une quelconque des revendications 1 à 9, dans lequel l'analyte est choisi dans le groupe constitué par les médicaments immunosuppresseurs, les médicaments contre les abus, l'acide folique et les vitamères de l'acide folique.
